# EUROPEAN PATENT APPLICATION

(11) **EP 3 657 176 A1**
(43) Date of publication of application: **27.05.2020**
(21) Application number: 18207954.1
(22) Date of filing: 23.11.2018
(51) Int. Cl.: G01N 33/68, C12Q 1/6883

(54) **DLK1 AS A PREDICTIVE MARKER FOR INSULIN RESISTANCE AND/OR TYPE 2 DIABETES AND/OR METABOLIC SYNDROME**

(71) Applicant: Syddansk Universitet, 5230 Odense M (DK)
(72) Inventor: Andersen, Ditte Caroline, 5250 Odensen SV (DK); Jensen, Charlotte Harken, 5250 Odense SV (DK); Sheikh, Søren Paludan, 2930 Klampenborg (DK)
(74) Representative: Plougmann Vingtoft a/s

(57) **Abstract**

The present invention relates to a method for predicting the risk of developing Insulin resistance and/or type 2 diabetes and/or metabolic syndrome for a subject, based on the levels of DLK1. A determined level above a reference level is indicative of said subject being at risk of developing Insulin resistance and/or type 2 diabetes and/or metabolic syndrome and a determined level equal to or below the reference level is indicative of said subject not being currently at risk of developing Insulin resistance and/or type 2 diabetes and/or metabolic syndrome.

## Description

### Technical field of the invention

The present invention relates to a method for predicting the risk of developing Insulin resistance and/or type 2 diabetes and/or metabolic syndrome for a subject. Said method is based on the level DLK1, wherein a level above a reference level is indicative of said subject being at risk of developing Insulin resistance and/or type 2 diabetes and/or metabolic syndrome.

### Background of the invention

Obesity has developed into one of the major burdens of modern societies and leads to numerous complications, including type 2 diabetes (T2D). More efficient anti-obesity strategies seem to require combination approaches, and identification of new drug targets is therefore warranted. Dysregulated expression of imprinted genes is known to have a marked impact on organism growth and development, but new evidence also suggests a postnatal effect where imprinted genes are implicated in human metabolic disorders. The Dlk1-Dio3 imprinted gene cluster is localized to mouse chromosome 12 and human chromosome 14, and defects in this cluster are associated with obesity, leanness, insulin resistance, and impaired glucose tolerance and metabolic disorders (Peters, 2014).
Delta-like 1 homolog (Dlk1) is one out of four protein- encoding paternally expressed genes in the Dlk1-Dio3 domain.

Previous transgenic mouse studies note that DLK1 inhibits adipose-tissue expansion (Moon et al. 2002, Lee et al. 2003; Villena et al 2008) and several clinical studies relating serum DLK1 levels to obesity/fat mass/weight gain show different relationships in humans affected by various diseases (Chacon et al., 2008; Kavalkova et al., 2013; Liangpunsakul et al., 2014).

Hence, an improved method for predicting the risk developing Insulin resistance and/or type 2 diabetes and/or metabolic syndrome would be advantageous, and in particular a more efficient and/or reliable method for early prediction of the risk developing Insulin resistance and/or type 2 diabetes and/or metabolic syndrome would be advantageous.

### Summary of the invention

The present invention shows that Dlk1, rather than acting as negative regulator of obesity, should be regarded as a factor linked to metabolic syndrome predisposition. The data further emphasizes the contributions from imprinted genes to metabolic disorders, and suggest Dlk1 as an anti-obesity target and a diagnostic tool for assessing predisposition for type 2 diabetes.

Thus, an object of the present invention relates to the provision of a biomarker for risk of developing Insulin resistance and/or type 2 diabetes and/or metabolic syndrome. In particular, it is an object of the present invention to provide a biomarker for early prediction of the risk of developing Insulin resistance and/or type 2 diabetes and/or metabolic syndrome.

Thus, one aspect of the invention relates to a method for predicting the risk of developing Insulin resistance and/or type 2 diabetes and/or metabolic syndrome for a subject, said method comprising
- determining (in vitro) in a biological sample from a subject the level of DLK1;
- comparing said level to a predetermined reference level;
wherein a determined level above said reference level is indicative of said subject being at risk of developing Insulin resistance and/or type 2 diabetes and/or metabolic syndrome and a determined level equal to or below said reference level is indicative of said subject not being at risk of developing Insulin resistance and/or type 2 diabetes and/or metabolic syndrome.

Another aspect of the present invention relates to a method for determining an increased, unchanged or decreased risk of developing Insulin resistance and/or type 2 diabetes and/or metabolic syndrome, the method comprising
- determining in a first sample from a subject the level of DLK1;
- determining in a second sample from the subject the level of DLK1, wherein said second sample has been obtained at a later time point than said first sample from said subject;
wherein, a determined DLK1 level in the second sample below the determined DLK1 level in the first sample is indicative of a lowered risk of developing Insulin resistance and/or type 2 diabetes and/or metabolic syndrome;
wherein, a determined DLK1 level in the second sample above the determined DLK1 level in the first sample is indicative of an increased risk of developing Insulin resistance and/or type 2 diabetes and/or metabolic syndrome; and
wherein, a determined DLK1 level in the second sample equal to the determined DLK1 level in the first sample is indicative of an unchanged risk of developing Insulin resistance and/or type 2 diabetes and/or metabolic syndrome.

Yet another aspect of the present invention is to provide a method for determining the effect of a treatment protocol against having or developing Insulin resistance and/or type 2 diabetes and/or metabolic syndrome, the method comprising
- determining in a first sample from a subject the level of DLK1;
- determining in a second sample from the subject the level of DLK1;
wherein, said second sample has been obtained at a later time point than said first sample from said subject and wherein the treatment protocol has been initiated or completed between the sampling of the first sample and the second sample; and
wherein, a determined DLK1 level in the second sample below the determined DLK1 level in the first sample is indicative of the treatment protocol being efficient against having or developing Insulin resistance and/or type 2 diabetes and/or metabolic syndrome; and
wherein, a determined DLK1 level in the second sample equal to or above the determined DLK1 level in the first sample is indicative of the treatment protocol not being efficient against having or developing Insulin resistance and/or type 2 diabetes and/or metabolic syndrome.

Still another aspect of the present invention is to provide a composition comprising a DLK1 inhibitory agent for use in the prevention or treatment of Insulin resistance and/or type 2 diabetes and/or metabolic syndrome.

A further aspect relates to the use of DLK1 as a biomarker for predicting for a subject the risk of developing Insulin resistance and/or type 2 diabetes and/or metabolic syndrome.

### Brief description of the figures

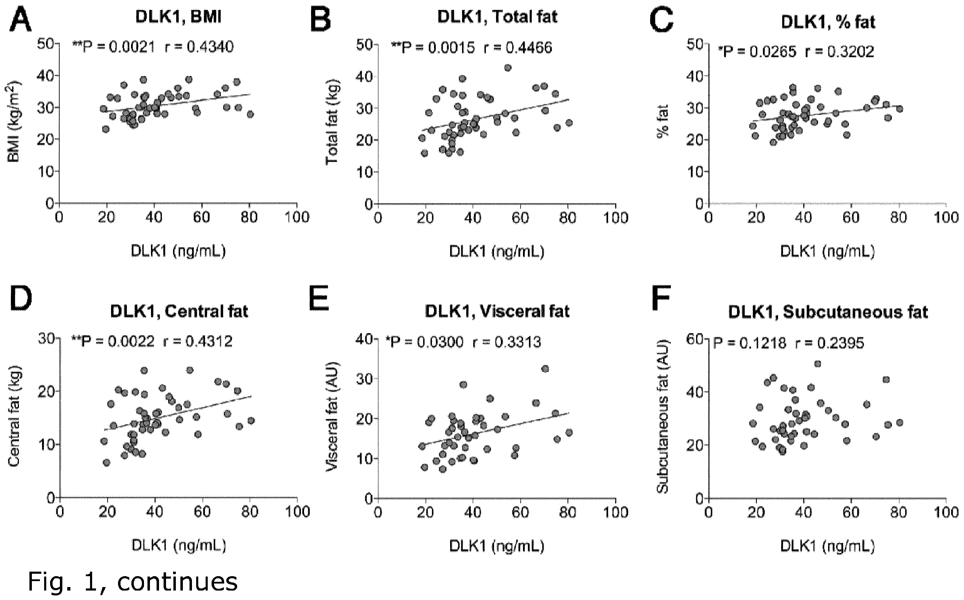

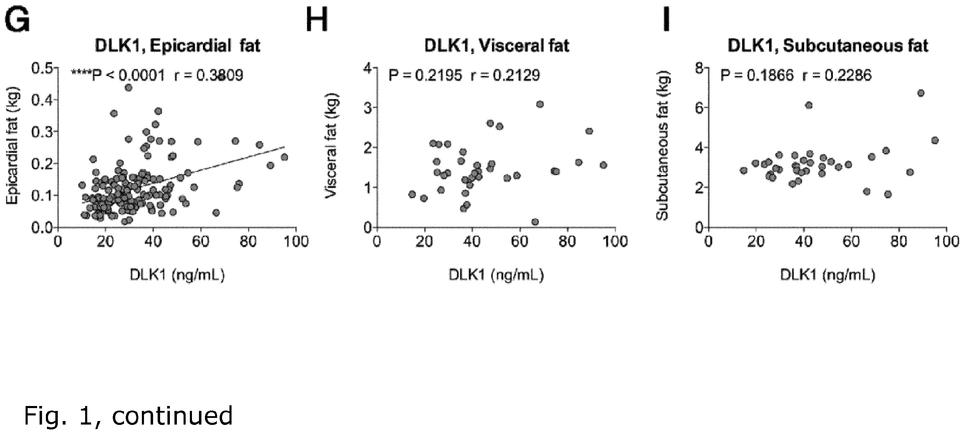

Figure 1 shows that DLK1 levels correlate with fat accumulation in humans. **(A-F)** Correlation analysis of circulating DLK1 in a Danish male cohort (n=48, median age 68 years, IQR 9; median BMI 29.8, IQR 5.5), with **(A)** BMI, **(B)** Total fat (kg), **(C)** Percentage of fat, **(D)** Central fat mass (kg), **(E)** Visceral fat mass (arbitrary unit (AU)=pixels*10⁻³), **(F)** subcutaneous fat mass (AU). **(G-I)** Correlation analysis of circulating DLK1 in an Italian cohort (n=158) consisting of non-diabetic men median age: 58 years IQR 21 and median BMI: 26.8, IQR 3.7, with **(G)** Epicardial fat amount, and for a subpopulation of n=35 (median age 47 years, IQR 15.1; median BMI 27.4, IQR, 4.3) **(H)** Visceral fat mass (kg) and **(I)** subcutaneous fat mass (kg). Data were tested for normality and analyzed using Spearman and Pearson correlation analysis, as appropriate.

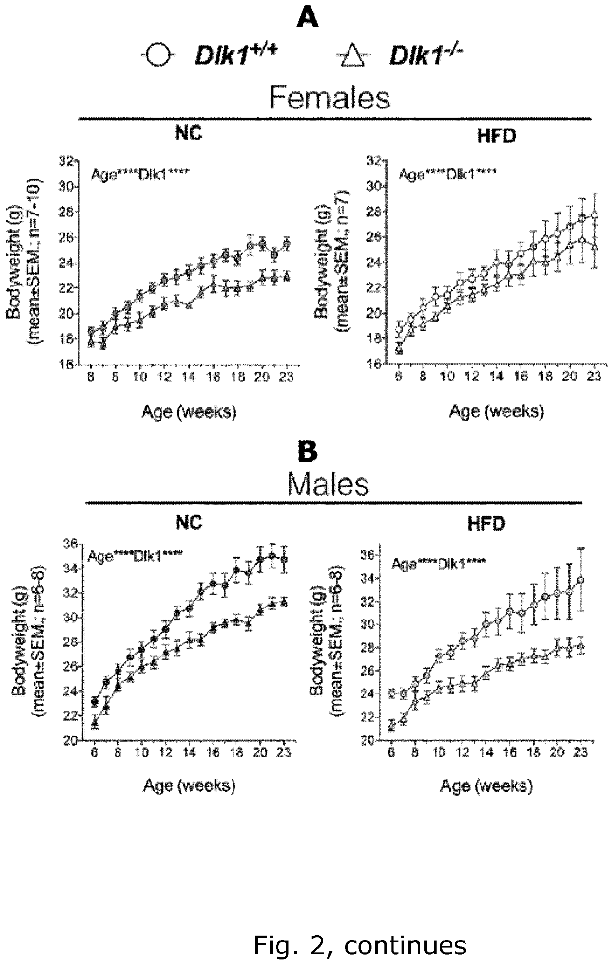

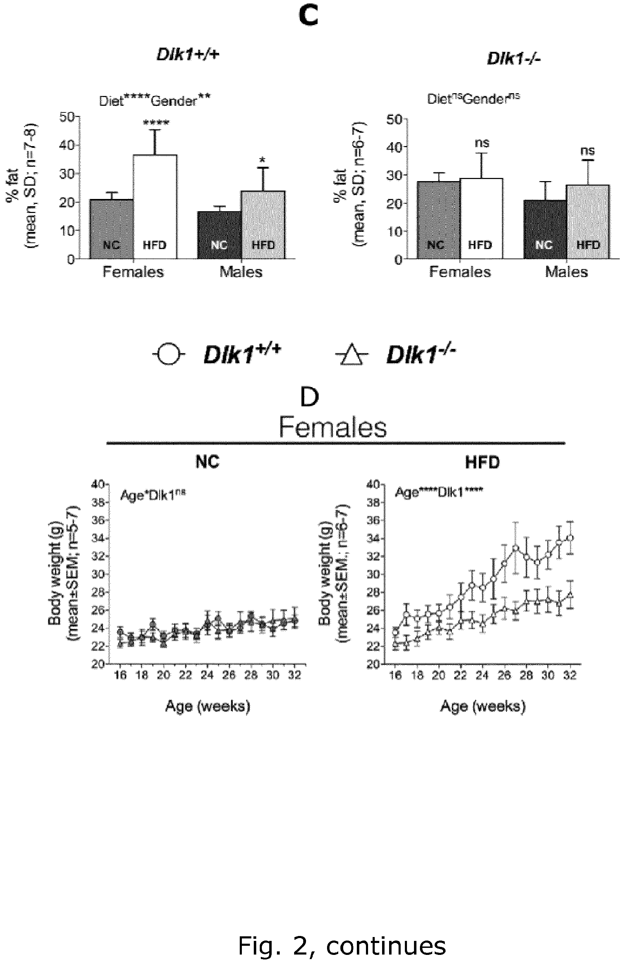

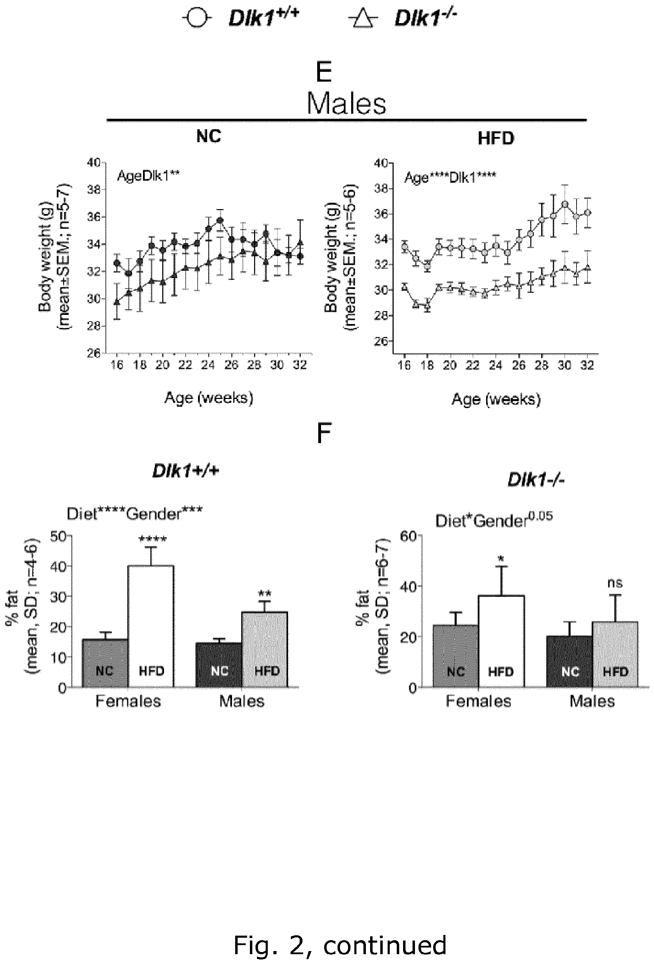

Figure 2 shows that Dlk1-/- mice weigh less than Dlk1+/+ mice regardless of the diet, and are resistant to HFD induced obesity. Six-week-old **(A-C)** and 16-week-old **(D-F)** Dlk1+/+ and Dlk1-/- mice were fed normal chow (NC) or high-fat diet (HFD, Altromin, 42% fat). Body weight was determined every week (A, B, D and E) and DXA scanning (diet age: 23 weeks; C and F) was performed to quantify percentage of body fat. Statistical difference was tested by a two-way ANOVA followed by Fisher's LSD test; *P<0.05, **P<0.01, ***P<0.001, ****P<0.0001, ns (not significant),**-o-**: Dlk1+/+; **-Δ-:** Dlk1-/-.

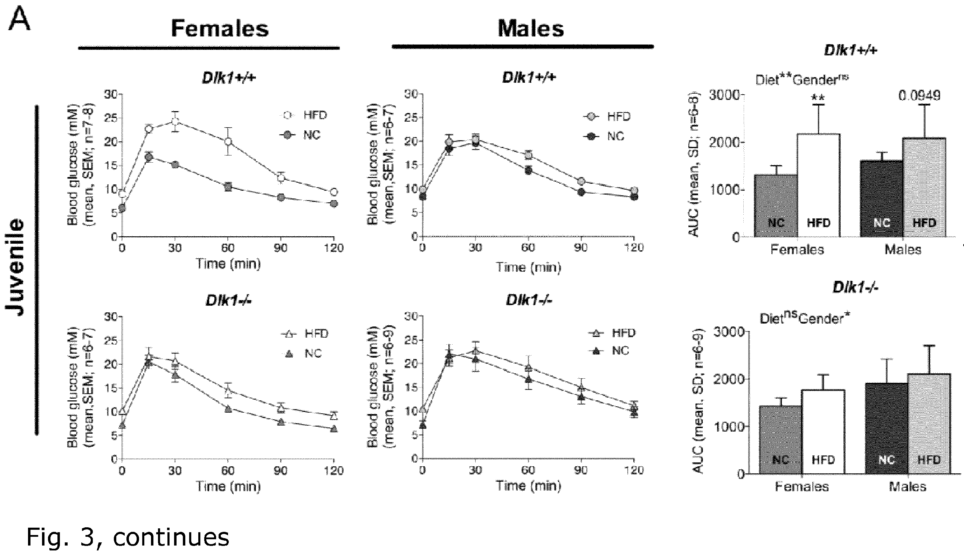

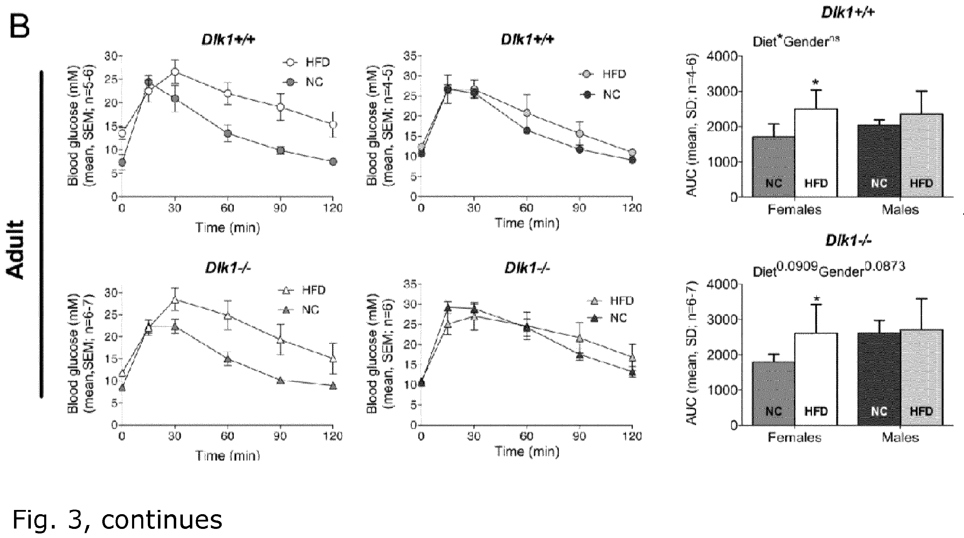

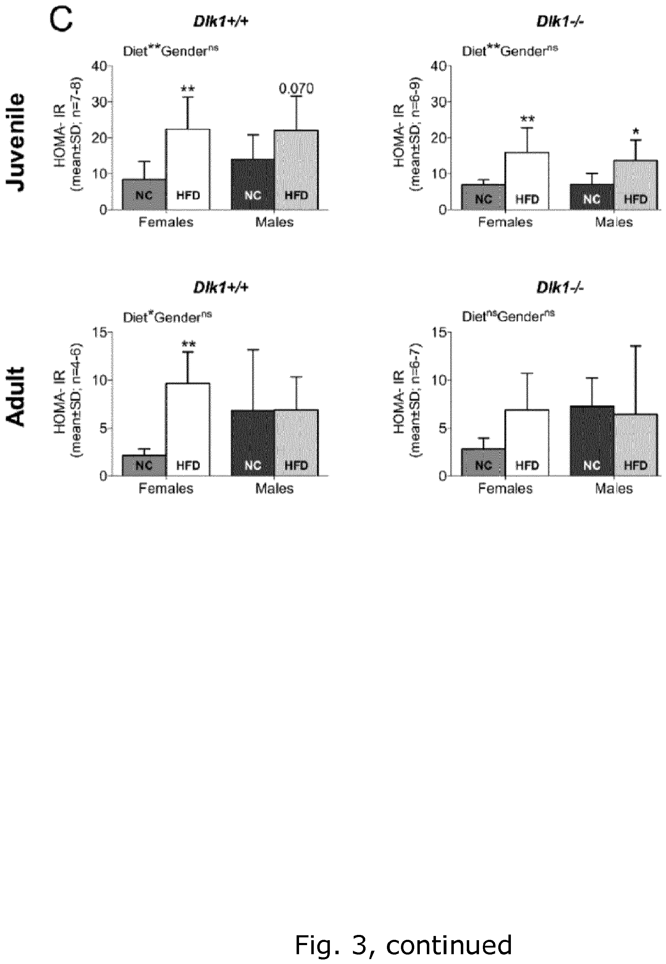

Figure 3 shows that loss of Dlk1 improves glucose homeostasis in female mice. **(A, C (Top))** Six-week-old or **(B, C (Bottom))** 16-week-old Dlk1+/+ and Dlk1-/- mice were fed normal chow (NC) or high-fat diet (HFD) for 25 weeks with Glucose tolerance tests (GTT) performed at the end of the experiment and fasting insulin and glucose levels measured to calculate the HOMA-IR index. For A-B, the area under the curve (AUC) was calculated for each mouse and used for statistical testing. In A-C, statistical difference was tested by a two-way ANOVA followed by Fisher's LSD test; *P<0.05, **P<0.01, ns (not significant).

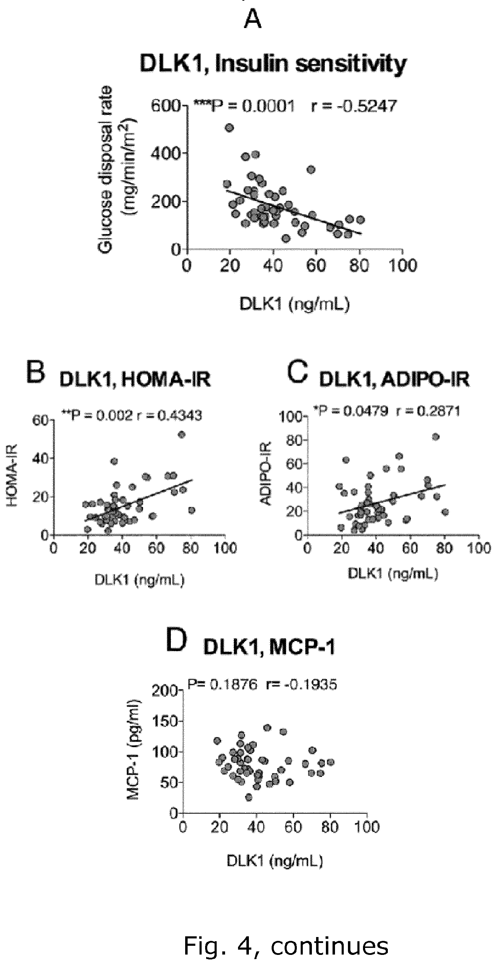

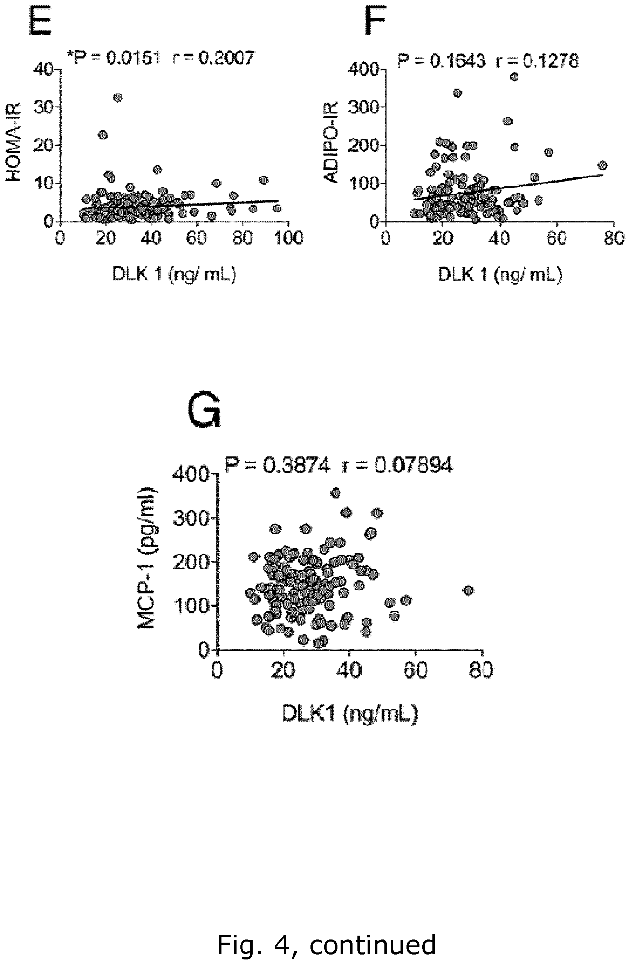

Figure 4 shows that Dlk1 is related to an adverse metabolic profile connected with insulin resistance in humans. Spearman correlation analysis of circulating DLK1 in 48 men (68-year-old, IQR 9; median BMI 30, IQR 5.6) from a Danish cohort with **(A)** glucose disposal rate, **(B)** HOMA-IR, **(C)** Adipo-IR, and **(D)** MCP-1. Spearman correlation analysis of circulating DLK1 in an Italian cohort of men (n=157; median age 58 years, IQR 21.; median BMI 26.8, IQR 3.7) with **(E)** HOMA-IR, **(F)** Adipo-IR, and **(G)** MCP-1.

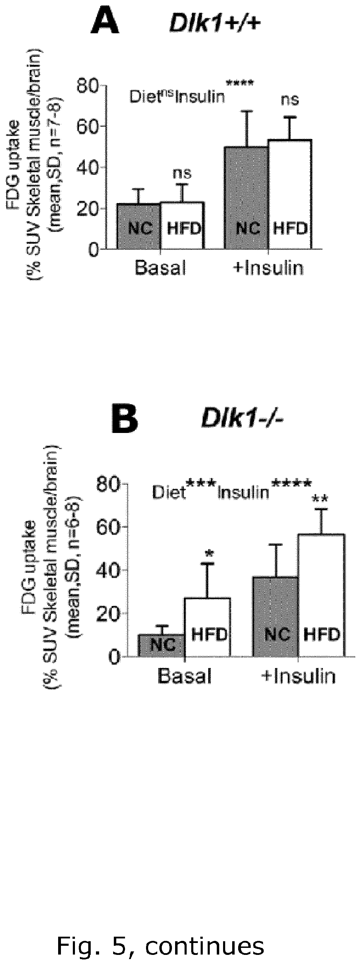

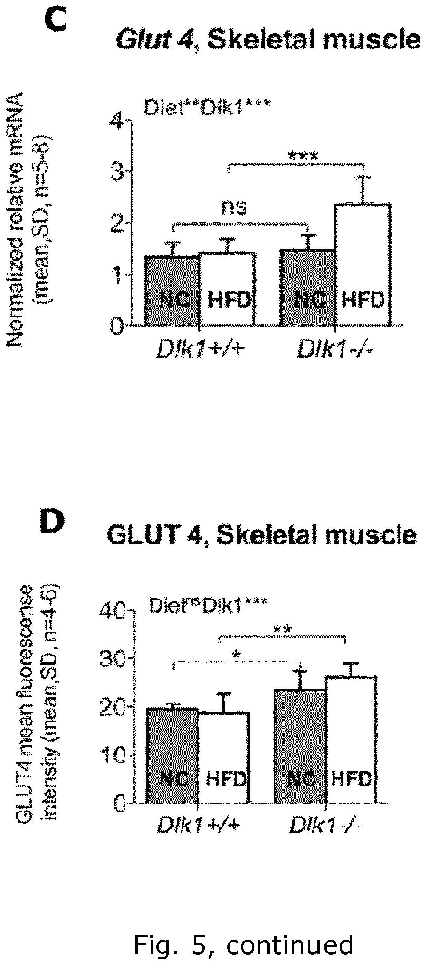

Figure 5 shows that lack of Dlk1 improves skeletal muscle glucose uptake. **(A-B)** Three-week-old Dlk1+/+ and Dlk1-/- female mice were fed NC or HFD short-term for 6 weeks and subjected twice (± insulin) to PET/CT scanning for determination of skeletal muscle glucose (18F-FDG) uptake. For each mouse, 18F-FDG standardized uptake value (SUV) for skeletal muscle was normalized to the brain SUV, and statistical difference was tested by a two-way ANOVA followed by Fisher's LSD test. **(C-D)** Six-week-old female Dlk1+/+ and Dlk1-/- mice were fed normal chow (NC) or high-fat diet (HFD) long-term (28 weeks), and **(C)** Glut4 (Slc2a4) mRNA levels were determined in hind limb (m. Gastrochnemius) skeletal muscles using qRT-PCR and **(D)** verified by GLUT4 quantification in immunohistochemical stainings. For qRT-PCR, raw data were normalized against Gapdh and beta-actin (qBASE- M:0.390; CV:0.137). For GLUT 4 IHC staining, mean intensity GLUT4 fluorescence was quantified in areas of both low and high GLUT4 to minimize subjectivity and an overall average was calculated for each animal. Statistical difference was tested by a two-way ANOVA followed by Fisher's LSD test. *P<0.05, **P<0.01, ***P<0.001, ****P<0.0001, ns (not significant).

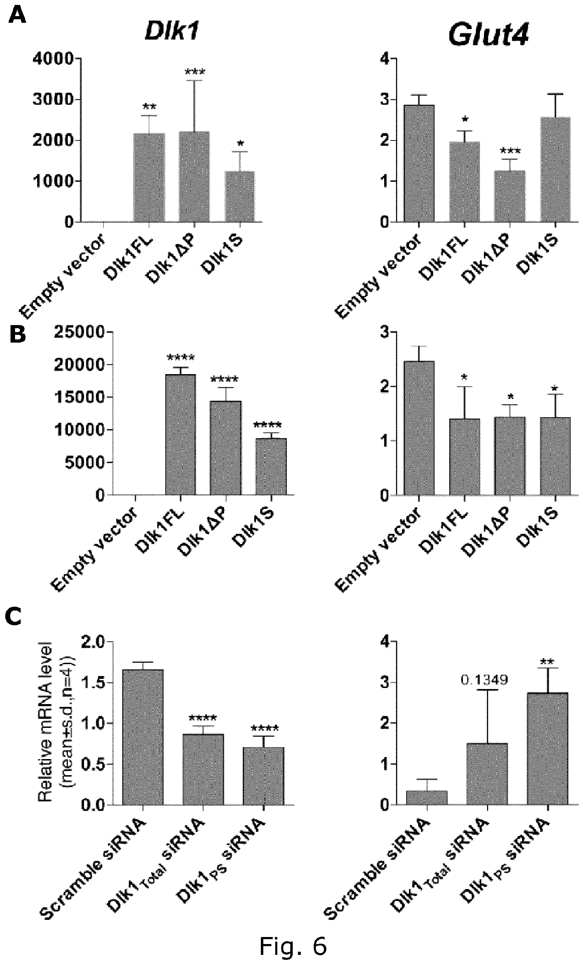

Figure 6 shows that in vitro Dlk1 overexpression and knock-down inhibits and enhances Glut4 expression, respectively. **(A, B)** Myoblasts were transfected with empty vector and different Dlk1 constructs (full-length Dlk1 (FL), Dlk1 lacking the protease recognition site (ΔP) and the extracellular soluble Dlk1 domain (S)) at two different concentrations before qPCR. **(C)** Dlk1 expression in 3T3-L1 preadipocytes that were transfected with siRNA-Scramble, or siRNAs against all Dlk1 isoforms (Dlk1Total) or Dlk1 isoforms containing the protease site (Dlk1PS) before differentiation and qPCR. For A-C, qPCR raw data were normalized against stably expressed control genes as determined by the Qbase-plus platform. Statistical difference was tested by a one-way ANOVA followed by Fisher's LSD test. *P<0.05, **P<0.01, ***P<0.001, ****P<0.0001.

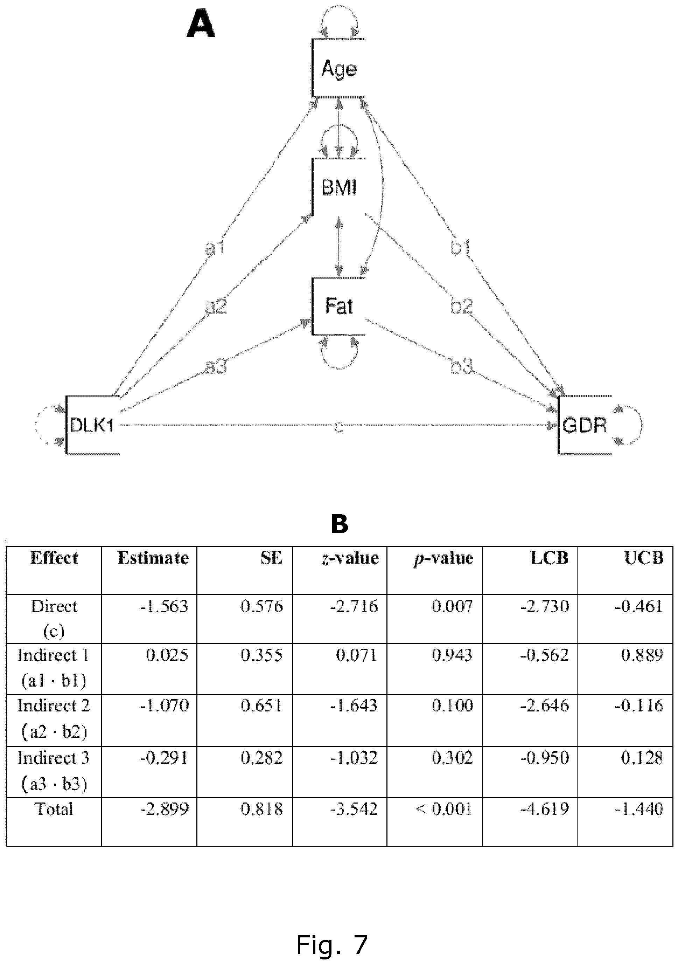

Figure 7 shows that the negative correlation between DLK1 and glucose disposal rate is not mediated by BMI, fat percentage or age, but is a direct and highly significant effect. **(A)** Path diagram of a multiple-mediator model in which the direct effect from DLK1 to GDR is labeled c. The effects from DLK1 to age, BMI, and fat percentage are labeled a1, a2, and a3, respectively. The effects from age, BMI, and fat percentage to GDR are labeled b1, b2, and b3, respectively. The three indirect effects explaining the extent to which the effect of DLK1 is mediated by age, BMI, and fat percentage are given by the products a1 · b1, a2 · b2, and a3 · b3, respectively. The total effect is given by the sum of direct and indirect effects: c + (a1 · b1) + (a2 · b2) + (a3 · b3).
Dependent variable: GDR. Independent variable: DLK1. Mediators: Age, BMI, and fat percentage. (B) Summary table of the mediation analysis. Estimates, standard errors, z-values, p-values, and 95% lower and upper confidence bounds (LCB and UCB, respectively). Bootstrapping with 5000 draws was used to estimate the standard errors.

The present invention will now be described in more detail in the following.

### Detailed description of the invention

### Definitions

Prior to discussing the present invention in further details, the following terms and conventions will first be defined:

### Delta-like 1 homolog (Dlk1)

Delta-like 1 homolog (Dlk1) is one out of four protein- encoding paternally expressed genes in the Dlk1-Dio3 domain. The DLK1 protein exists in transmembrane and shed forms and is highly homologous to the Notch-Delta protein family.

### Reference level

In the context of the present invention, the term "reference level" relates to a standard in relation to a quantity, which other values or characteristics can be compared to.

In one embodiment of the present invention, it is possible to determine a reference level by investigating the abundance of the biomarker according to the invention in (blood e.g. serum) samples from e.g. healthy subjects. In another embodiment, the reference levels are obtained from diseased subjects. Of course, the reference must then be adjusted accordingly.

Based on these results, a cut-off may be obtained that shows the relationship between the level(s) detected and patients at risk. The cut-off can thereby be used to determine the amount of the one or more biomarkers, which corresponds to for instance an increased risk of developing metabolic syndrome and/or Insulin resistance (IR) and/or type 2 diabetes.

### Risk Assessment

The present inventors have successfully developed a new method to predict the risk for developing metabolic syndrome and/or Insulin resistance (IR) and/or type 2 diabetes for a subject. The results presented in the examples show that the described biomarker appear to be an efficient biomarker for determining whether a patient has an increased risk of developing Insulin resistance and/or type 2 diabetes and/or metabolic syndrome.

To determine whether a patient has an increased risk of developing Insulin resistance and/or type 2 diabetes and/or metabolic syndrome a cut-off must be established. This cut-off may be established by the laboratory, the physician or on a case-by-case basis for each patient.

The cut-off level could be established using a number of methods, including: multivariate statistical tests (such as partial least squares discriminant analysis (PLS-DA), random forest, support vector machine, etc.), percentiles, mean plus or minus standard deviation(s); median value; fold changes.

The multivariate discriminant analysis and other risk assessments can be performed on the free or commercially available computer statistical packages (SAS, SPSS, Matlab, R, etc.) or other statistical software packages or screening software known to those skilled in the art.

As obvious to one skilled in the art, in any of the embodiments discussed above, changing the risk cut-off level could change the results of the discriminant analysis for each subject.

Statistics enables evaluation of the significance of each level. Commonly used statistical tests applied to a data set include t-test, f-test or even more advanced tests and methods of comparing data. Using such a test or method enables the determination of whether two or more samples are significantly different or not.

The significance may be determined by the standard statistical methodology known by the person skilled in the art.

The chosen reference level may be changed depending on the mammal/subject for which the test is applied.

Preferably, the subject according to the invention is a human subject, such as a subject considered at risk of having or developing Insulin resistance and/or type 2 diabetes and/or metabolic syndrome.

The chosen reference level may be changed if desired to give a different specificity or sensitivity as known in the art. Sensitivity and specificity are widely used statistics to describe and quantify how good and reliable a biomarker or a diagnostic test is. Sensitivity evaluates how good a biomarker, or a diagnostic test is at detecting a disease, while specificity estimates how likely an individual (i.e. control, patient without disease) can be correctly identified as not sick.

Several terms are used along with the description of sensitivity and specificity; true positives (TP), true negatives (TN), false negatives (FN) and false positives (FP). If a disease is proven to be present in a sick patient, the result of the diagnostic test is considered to be TP. If a disease is not present in an individual (i.e. control, patient without disease), and the diagnostic test confirms the absence of disease, the test result is TN. If the diagnostic test indicates the presence of disease in an individual with no such disease, the test result is FP. Finally, if the diagnostic test indicates no presence of disease in a patient with disease, the test result is FN.

### Sensitivity

Sensitivity = TP/ (TP + FN) = number of true positive assessments/ number of all samples from patients with disease.

As used herein, the sensitivity refers to the measures of the proportion of actual positives, which are correctly identified as such - in analogy with a diagnostic test.

### Specificity

Specificity= TN/ (TN+ FP) = number of true negative assessments/ number of all samples from controls.
As used herein, the specificity refers to measures of the proportion of negatives, which are correctly identified. The relationship between both sensitivity and specificity can be assessed by the ROC curve. This graphical representation helps to decide the optimal model through determining the best threshold -or cut-off for a diagnostic test or a biomarker candidate.

As will be generally understood by those skilled in the art, methods for screening are processes of decision-making and therefore the chosen specificity and sensitivity depend on what is considered to be the optimal outcome by a given institution/clinical personnel.

It would be obvious for a person skilled in the art that it may be advantageous to select a higher sensitivity at the expense of lower specificity in most cases, to identify as many patients with disease risk as possible.
In a preferred embodiment, the invention relates to a method with a high specificity, such as at least 70%, such as at least 80%, such as at least 90%, such as at least 95%, such as 100%. In another preferred embodiment, the invention relates to a method with a high sensitivity, such as at least 80%, such as at least 90%, such as 100%.

### Method for predicting the risk of developing Insulin resistance and/or type 2 diabetes and/or metabolic syndrome

As also outlined above, the present invention suggests that Dlk1, rather than acting as negative regulator of obesity, is as a factor linked to metabolic syndrome predisposition. The data further suggest Dlk1 as an anti-obesity target and a diagnostic tool for assessing predisposition for insulin resistance and type 2 diabetes. Thus, a first aspect of the invention relates to a method for predicting the risk of developing Insulin resistance and/or type 2 diabetes and/or metabolic syndrome, said method comprising
- determining in a biological sample from a subject the level of DLK1;
- comparing said level to a predetermined reference level;
wherein a determined level above said reference level, is indicative of said subject being at risk of developing Insulin resistance and/or type 2 diabetes and/or metabolic syndrome and a determined level equal to or below said reference level is indicative of said subject not being at risk of developing Insulin resistance and/or type 2 diabetes and/or metabolic syndrome.
In example 2, it is shown that DLK1 levels are associated with increased body fat in humans, thus contradicting Dlk1 being an inhibitor of obesity. Example 3 confirms these results in DLK1 knockout mice. Example 4 indicates that increased plasma DLK1 is an early predictor of insulin resistance and TD2 (type 2 diabetes). Example 5 shows that lack of Dlk1 improves skeletal muscle glucose uptake by increased expression of the major glucose transporter GLUT4. Example 6 points to Dlk1 being a regulator of GLUT4 mediated glucose uptake, partially explaining why high fat diet-fed *Dlk1*^{-/-} animals are less obese and insulin resistant as compared with *Dlk1*^{+/+} mice. Example 7 shows by testing in a multiple-mediator model, that the negative correlation of serum DLK1 and glucose disposal rate is not indirectly mediated by age, BMI or fat percentage.

Thus, in an embodiment the method is for predicting the risk of developing metabolic syndrome. In another embodiment, the method is for predicting the risk of developing Insulin resistance (IR). In yet an embodiment, the method is for predicting the risk of developing type 2 diabetes (TD2).

MCP-1 is an early marker of inflammation. General inflammation plays a crucial role in the development of metabolic syndrome/T2DM and elevated MCP-1 levels have been reported to predict later glucose dysregulation. In an embodiment, said subject has a blood serum level of MCP-1 in a normal range showing absence of inflammation and no established type 2 diabetes. As shown in example 4, MCP-1 levels did not correlate with DLK1 levels, nor did MCP-1 correlate with insulin sensitivity (not shown). Thus, DLK1 appears to be an earlier biomarker for metabolic syndrome and/or Insulin resistance (IR) and/or type 2 diabetes than MCP-1.

Metabolic syndrome can be defined as being caused by a combination of different medical conditions. Thus, in an embodiment, the metabolic syndrome is predicted by at least three of the five following medical conditions: abdominal obesity (in specific)/obesity (in general), high blood pressure, high blood sugar, high serum triglycerides and low high-density lipoprotein (HDL) levels.

In the present context abdominal obesity is defined either by general overweight BMI (>25) or by waist circumference >94cm (Males) and >80cm (Females).
In the present context high blood pressure is defined as >140/90mmHg.
In the present context high blood sugar is defined as >6.0mmol/L.
In the present context high serum triglycerides is defined as >1.7mmol/L.
In the present context low high-density lipoprotein (HDL) levels is defined as <1.03mmol/L (Males) and 1.29 mmol/l (Females).

The biological sample can be selected from different types of biological samples. Thus, in an embodiment, the biological sample is selected from the group consisting of a blood sample, such as whole blood, serum and/or plasma, saliva, and urine, preferably serum or plasma. In the example section, blood serum or plasma has been tested.

The level of DLK1 can be determined in different ways. Thus, in an embodiment, the level of DLK1 is determined at the mRNA level and/or the protein level, preferably the protein level. In yet an embodiment, the protein level is determined by ELISA, RIA, 1H-NMR and/or mass spectrometry.

A reference level can be selected/determined in different ways. Thus, in an embodiment the predetermined reference value is based on an average of DLK1 levels in a control population not considered at risk of having Insulin resistance and/or type 2 diabetes and/or metabolic syndrome, a control population considered of having Insulin resistance and/or type 2 diabetes and/or metabolic syndrome and/or (a) sample(s) from the same subject previously obtained in time. In an embodiment, the reference level is above 20 ng/ml in blood serum, such as above 30 ng/ml, such as above 40 ng/ml, such as above 50 ng/ml, preferably such as above 60 ng/ml, more preferably such as above 70 ng/ml, or such as in the range 20-100 ng/ml, such as in the range 40-80 ng/ml or preferably such as in the range 60-80 ng/ml in blood serum, and more preferably in the range 70-80 ng/ml. Example 2 shows measured levels of Dlk1 in blood serum, indicating that serum levels in the above intervals may be a relevant reference level using a previously described quantitative DLK1 assay (DLK1 ELISA) (Jensen et al. Fetal antigen 1 (FA1), a circulating member of the epidermal growth factor (EGF) superfamily: ELISA development, physiology and metabolism in relation to renal function. Clin Chim Acta 1997;268:1-20). Other reference values and units (including arbitrary units) may apply when using alternative assays for quantification of DLK1. Independent of DLK1 assay, reference values may vary with gender and/or age as also known to the skilled person.
In yet an embodiment, the subject is a mammal such as a human; a non-human species, including a primate; a livestock animal such as a sheep, a cow, a pig, a horse, a donkey, or a goat; a laboratory test animals such as mice, rats, rabbits, guinea pigs, or hamsters; or a companion animal such as a dog or a cat, preferably the subject is a human.

In a related embodiment, the subject is a male or a female.

In yet an embodiment, the method is used to devise a stratified diet for a group of subjects or a personalized diet for the subject.

### Evaluating progression of risk

In another aspect, the invention relates to a method for determining an increased, unchanged or decreased risk of developing Insulin resistance and/or type 2 diabetes and/or metabolic syndrome, the method comprising
- determining in first sample from a subject the level of DLK1;
- determining in second sample from the subject the level of DLK1, wherein said second sample has been obtained at a later time point than said first sample from said subject;
wherein, a determined DLK1 level in the second sample below the determined DLK1 level in the first sample is indicative of a lowered risk of developing Insulin resistance and/or type 2 diabetes and/or metabolic syndrome;
wherein, a determined DLK1 level in the second sample above the determined DLK1 level in the first sample is indicative of an increased risk of developing Insulin resistance and/or type 2 diabetes and/or metabolic syndrome; and
wherein, a determined DLK1 level in the second sample equal to the determined DLK1 level in the first sample is indicative of an unchanged risk of developing Insulin resistance and/or type 2 diabetes and/or metabolic syndrome.

### Evaluating treatment protocol

In a further aspect, the invention relates to a method for determining the effect of a treatment protocol against having or developing Insulin resistance and/or type 2 diabetes and/or metabolic syndrome, the method comprising
- determining in first sample from a subject the level of DLK1;
- determining in second sample from the subject the level of DLK1;
wherein, said second sample has been obtained at a later time point than said first sample from said subject and wherein the treatment protocol has been initiated or completed between the sampling of the first sample and the second sample; and
wherein, a determined DLK1 level in the second sample below the determined DLK1 level in the first sample is indicative of the treatment protocol being efficient against having or developing Insulin resistance and/or type 2 diabetes and/or metabolic syndrome; and
wherein, a determined DLK1 level in the second sample equal to or above the determined DLK1 level in the first sample is indicative of the treatment protocol not being efficient against having or developing Insulin resistance and/or type 2 diabetes and/or metabolic syndrome.

In an embodiment, the treatment protocol is selected from the group consisting of a medical treatment, exercise and a switch in diet, such as a diet promoting weight loss.

### Use of DLK1 as a biomarker

In yet an aspect, the invention relates to the use of DLK1 as an (in vitro) biomarker for predicting a subject's risk of developing Insulin resistance and/or type 2 diabetes and/or metabolic syndrome.

### Treatment

Yet an additional aspect of the invention relates to a composition comprising a DLK1 inhibitory agent for use in the prevention or treatment of Insulin resistance and/or type 2 diabetes and/or metabolic syndrome.

In an embodiment, the DLK1 inhibitory agent is selected from the group consisting of DLK1 binding agents, such as selected from the group consisting of a polyclonal antibody, a monoclonal antibody, an antibody, wherein the heavy chain and the light chain are connected by a flexible linker, an Fv molecule, an antigen binding fragment, a Fab fragment, a Fab' fragment, a F(ab')₂ molecule, a fully human antibody, a humanized antibody, and a chimeric antibody.

In a related embodiment the DLK1 inhibitory agent is an agent inhibiting the translation of DLK1 RNA to protein, such as a siRNA, miRNA and antisense or by inhibiting the transcription of the *DLK1* gene by targeting the promotor region (promotor-specific transcription inhibition).

It should be noted that embodiments and features described in the context of one of the aspects of the present invention also apply to the other aspects of the invention.

All patent and non-patent references cited in the present application, are hereby incorporated by reference in their entirety.

The invention will now be described in further details in the following non-limiting examples.

### Examples

### Example 1 - Materials and methods

### Study populations

56 women were subdivided into obese (median BMI: 41.3; Inter Quartile Range, IQR, 11.8; n=30) and non-obese (BMI: 24.1 (IQR, 2.78; n=26) with a median age of 41.5 (IQR, 12).

Subjects from one Italian cohort included 35 healthy non-diabetic men with median age 47 years (IQR, 15.1) and median BMI of 27.4 (IQR, 4.3) which all underwent Magnetic resonance imaging (MRI) scans. Another Italian cohort, examined by Computer Tomography (CT) and with available data on their epicardial fat, consisted of 158 non-diabetic men, with median age 62 years (IQR, 18) and median BMI of 26.5 (IQR, 3.4).

A Danish cohort encompassed 48 healthy non-diabetic men with a median age of 68 years (IQR, 9) and a median BMI of 30.0 (IQR, 5.6). Six patients from the initial population of 54 subjects were excluded based on a diabetic MCP-1 value (n=2), DLK1 levels >100 (ng/mL)(n=1), missing measures (n=2), and hemolysis in blood sample (n=1). Dual X-ray absorptiometry (DXA) was used to measure percentage of body fat (n=48) and quantification of visceral and subcutaneous fat was performed by MRI (n=43). Euglycemic hyperinsulinemic clamps (n=48) were performed as follows: Briefly, after a 120-min. basal tracer equilibration period, insulin (Actrapid; Novo Nordisk, DK) was infused at a rate of 40 mU/m2/min. for 180 min leading to physiological hyperinsulinemia at approximately 400 pmol/l in the insulin-stimulated period. Plasma glucose levels were clamped at approximately 5 mmol/l, using a variable infusion rate of 20% glucose based on bedside plasma glucose measurements (ABL800 Flex, Radiometer, DK) every 10-20 min. Mean glucose disposal rate (mg/min/m²) at steady state (the last 20 min. of clamp) was taken as an estimate of whole-body insulin sensitivity.

### Animals and diets

Dlk1-/- and Dlk1+/+ colonies were generated and maintained by homozygous breeding and backcrossing. Animals were fed ad libitum with a normal chow (NC; 10% fat, 20% protein, 70% carbohydrate) or high-fat diet (HFD; 45% fat, 35% carbohydrate, 20% protein) (Brogaarden, Denmark). For all animal experiments, age- and gender-matched animals were used as indicated.

### Mouse and human plasma measurements

Blood from Dlk1-/- and Dlk1+/+ animals was collected weekly from the tail vein for 16-17 weeks, and DLK1 levels were measured using a mouse DLK1 ELISA with two monoclonal antibodies (CC-5 and CC-11, generated in-house) against mouse DLK1 were used as catcher/detection antibodies. Plasma levels of Monocyte Chemoattractant Protein-1 (MCP-1) were measured by ELISA. Glucagon was measured by ELISA as recommended by manufacturer (Mercodia).

### Glucose tolerance test

A Glucose Tolerance Test (GTT) was performed in Dlk1-/- and Dlk1+/+ males/females fed HFD or NC. Briefly, animals were fasted for 6 hours and following an intraperitoneal (i.p.) injection of D-Glucose (2g/kg body weight), blood glucose levels were monitored at indicated time points using the One Touch Ultra® system (LifeScan).

In an immunoneutralization experiment, Dlk1+/+ animals (n=15) fed HFD for 13 weeks were injected i.p. with a mouse monoclonal antibody (IgG1, k, in house) recognizing an epitope on the extracellular part of the mouse DLK1 protein, or with an isotype-matched control antibody (10 µg MAb/g body weight). GTT was performed one and three days after injection.

### Homeostasis Model Assessment for Insulin Resistance

The homeostasis model assessment method was used to calculate insulin resistance (HOMA-IR), from fasting plasma glucose and insulin levels: HOMA-IR = (FPI × FPG)/22.5, where FPI is the fasting plasma insulin concentration (mU/L) and FPG is the fasting plasma glucose level (mmol/L). Adipo-IR, a measure of adipose tissue insulin resistance = Fasting FFAs (mM) × Insulin (pM).

### Body composition (DXA)

Body composition, including total fat mass, was determined by dual energy X-ray absorptiometry (DXA) using the Lunar PIXImus Mouse Densitometer (Wipro GE Healthcare, WI) with the mice under inhalation anesthesia using 2% Isoflurane.

Small animal positron emission-/computer- tomography (PET/CT) imaging Small-animal PET/CT was performed on a Siemens INVEON multimodality preclinical scanner. Mice (Dlk1+/+ n=15, Dlk1-/- n=13) were fasted overnight prior to PET/CT scanning at baseline (day 0) and one day later (day 1). Mice were subcutaneously injected with 75 µl saline or 0.75 U/(Kg body weight) short acting insulin (Humulin, NOVO Nordisk, DK) respectively, 15 min. before injection of 18F-Fluoro-deoxy-glucose (FDG). Mice were anesthetized (2% isoflurane), given a bolus injection of FDG (day 0, 95.5±9.1 MBq. Day 1, 91.6±13.4 MBq) via a tail vein catheter. Prior to the PET scan a two-bed CT scan was performed for attenuation correction of the PET data and anatomic orientation. CT and PET images were co-registered using a transformation matrix and CT-based attenuation correction was applied to the PET data. The PET data was reconstructed using the Siemens INVEON pre-clinical software and data analysis of the PET/CT fused images was performed with INVEON software version 4.2 (IRW, Siemens). FDG uptake in each volume of interest (VOI) was reported as standardized uptake values (SUV). VOIs of the skeletal limb hind muscles were normalized using brain uptake as a reference structure that metabolizes glucose independent of insulin.

### In vitro cell transfections

Mouse C2C12 myoblast as well as 3T3-L1 preadipocytes were cultured. For plasmid transfection we used vectors expressing the entire DLK1 protein (pLHDLK1-HA), protease site-lacking DLK1 (pLHDLK1ΔP-HA), soluble DLK1 (pLHDLK1E-HA) or empty vector (pLHCX-HA). Plasmids were purified using a QIAGEN Plasmid Miniprep Kit (Qiagen) and by Nanodrop (Saveen Werner, DK) whereas purity was evaluated by visualization on a 1% agarose gel. Transfections were performed using electroporation (Amaxa Nucleofector™ Technology, Lonza) at optimal confluence according to the manufacturer's instructions. Myoblasts (2.7-3.7x106) were suspended in 100 µl of Cell line Mirus Nucleofector solution (Lonza) with different two different concentrations of plasmids reaching 10-fold expression of DLK1. Cells were analyzed by immunostaining or qRT-PCR. For preadipocytes, siRNA transfections were performed.

### qPCR and mRNA arrays

Relative expression levels were obtained after normalizing the raw data against multiple endogenous control genes as determined by the qBase+ platform (Biogazelle). In the Swedish cohort, total RNA was isolated from subcutaneous abdominal adipose tissue biopsies. Biotinylated complementary RNA was subsequently analyzed using the GeneChip Human Gene 1.0 ST Array (Affymetrix Inc., Santa Clara, CA) and standardized protocols.

### Adipocyte size quantification and Immuno(cyto/histo)chemistry

Average adipocyte volume (pL) was determined using HE-stained sections of formaldehyde fixed (FFPE) gonadal adipose tissue obtained from six weeks old Dlk1+/+ (n=12), Dlk1-/- (n=12) mice fed NC or HFD for 28 weeks before sacrificed. Two sections (each comprising five fields) from each animal were used for quantification. Images were acquired using a Leica DMI4000B Cool Fluo Package instrument equipped with a Leica DFC340 FX Digital Camera and analyzed using ImageJ 1.48 software with MRI adipocyte size tool. Average adipocyte volume was calculated from the Feret's diameter values. Immunohistochemistry of FFPE tissues was performed with rabbit anti-mouse DLK1 (1:2,000; in-house) or rabbit anti-Glut4 (1:1000; ab33780, Abcam) antibodies. Secondary antibodies used were Alexa 555- or 488-conjugated donkey anti-IgG (1:200, Molecular Probes), and mounting medium contained DAPI (Vectashield, Vector Labs). Images were captured using a Leica DMI4000B instrument equipped with a Leica DFC340 FX Digital Camera. Exposure and picture processing were applied equally to m.gastrocnemius sample sections and controls. GLUT 4 protein expression differed within all specimens with some areas showing high membrane appearance, whereas others having much less membrane GLUT4 localization. To minimize subjectivity in GLUT4 quantification, a picture was acquired for all cross sectional areas (high and low) in a given specimen. Analysis was performed using the ImageJ software, where ROIs were used to remove areas such as large vessels and mesenchyme. For each specimen, two averages of mean intensity were calculated for high- (n=6) and low (n=4-6, 3 specimens did not include low intensity areas of cross section) intensity areas, and a final average (high and low (n=4-6)) of the entire section was calculated.

### Statistical analyses

Statistical analyses were performed as indicated either by One or Two-way ANOVA (Sidak's multiple comparisons posttest or Fisher's LDS test) or by two-tailed t-tests. Clinical values were presented using the median and interquartile range (IQR). Associations between DLK1 and clinical parameters of interest were evaluated by Spearman Rank correlation analysis after testing for data normality or by Pearson of logarithmic transformed data. Outliers were removed by GraphPad Prism. As indicated, data were corrected for confounding using multivariate analysis. For statistical analysis, we used the R software (version 3.2.1) and GraphPad Prism (version 6).

### Study approval

All animal studies were approved by the Danish Council for Supervision with Experimental Animals (no. 2012-15-2934-00313). The human studies were approved by the respective national ethics committees in Denmark. All participants gave written informed consent before inclusion in the original cohorts.

### Example 2 - DLK1 levels correlates with increased body fat in humans

### Aim of study

To evaluate the correlation between DLK1 levels and body fat in humans

### Materials and methods

See also example 1 for details.

### Results

Circulating DLK1 levels were measured in blood samples from healthy to overweight, non-diabetic Danish men (68 years (9 (median; IQR)); n=52) with a median BMI of 29.8 (5.4)). The median concentration of serum DLK1 was 36.15 ng/ml (IQR:17.2) and correlated positively with BMI (Fig. 1A).

In contrast to what could have been expected from previous cell- and animal studies, there was no negative association between DLK1 levels and the amount of fat as determined by Magnetic Resonance Imaging (MRI) (Fig. 1B-F). In fact, the absolute amount of fat as well as the percentage of fat increased significantly with increased levels of circulating DLK1 (Fig. 1B-C). The relative amount of abdominal fat is in general associated with increased risk of developing the metabolic syndrome. It was found that increased levels of circulating DLK1 was associated to increased amounts of central fat mass (Fig. 1D), and this seems to be a consequence of increased amounts of visceral-, but not subcutaneous fat (Fig. 1E-F).

Increased epicardial fat is another marker of the metabolic syndrome. In a population of 158 healthy, non-diabetic middle-aged Italian men (median age: 58 years (21 (IQR) and median BMI: 26.8 (3.7)), the median concentration of serum DLK1 was 28.9 ng/ml (17.1) and correlated positively with BMI (**P=0.0068; Spearman r = 0.2143). In this population, the amount of epicardial fat was significantly associated with DLK1 levels (Fig. 1G), further supporting a link between DLK1 and increased fat in humans as well as the metabolic syndrome. No correlations were observed between DLK1 and visceral or subcutaneous fat amounts (Fig.1H-I) in this population.

### Conclusion

These unexpected data suggest, that in normal to overweight healthy human subjects, DLK1 levels are associated with increased body fat, thus contradicting Dlk1 being an inhibitor of obesity, in normal to overweight humans at least.

Thus, DLK1 levels can be seen as a predictive marker for the risk of developing metabolic syndrome and/or Insulin resistance and/or type 2 diabetes.

### Example 3 - Dlk1 knockdown in mice limits body weight irrespective of diet and age, and protects against diet-induced obesity.

### Aim of study

To further investigate the unexpected positive correlations between increased DLK1 and fat, by the use of a Dlk1-/- mouse strain.

### Results

Adult Dlk1-/- mice were completely negative for Dlk1 in all examined tissues, whereas Dlk1+/+ mice expressed Dlk1 in specific tissues.

As a generally accepted model for the induction of obesity, we compared Dlk1+/+ and Dlk1-/- mice fed a normal chow (NC) or a high-fat diet (HFD) starting at the age of 6 weeks (= puberty in mice) (Fig. 2A-C). In both genders, and irrespective of diet, a significantly reduced weight of Dlk1-/- mice was found compared with Dlk1+/+ control mice (Fig. 2A), but the percentage of weight gain on NC and HFD was similar between genotypes (data not shown). To obtain accurate measures of fat content in these mice, a DXA scanning was used. At basal levels (NC), the %fat was slightly increased in Dlk1-/- as compared to Dlk1+/+ at NC for both genders (Figure 2C). However, whereas Dlk1+/+ animals increased their fat percentages substantially upon HFD diet, Dlk1-/- animals were resistant, and did not change their %fat content (Figure 2C). It was found that Dlk1+/+ animals independent of gender exhibited an increase in fat cell volume upon HFD, whereas Dlk1-/- animals showed no change despite having slightly enlarged adipocytes at NC as compared to Dlk1+/+ animals (data not shown). Also, a negative association between DLK1 mRNA and fat cell size in WAT subcutaneous biopsies obtained from normal to overweight human subjects was not found (Data not shown).

Since the adipose tissue develops during late postnatal and juvenile life, the results of an early postnatal HFD feeding protocol as tested above may have influenced fat tissue development in itself.

Thus, next it was tested if the absence of Dlk1 affected fat expansion in adult animals by performing a new diet study starting at an age of 16 weeks. In contrast to the findings in juvenile animals (Fig. 2AB), the difference in body weight between genotypes in NC fed mice, particularly in females, virtually disappeared (Fig. 2DE). For females on HFD, this resulted in a substantially lower weight gain (Data not shown). As for the juvenile animals, DXA scanning showed a slightly increased fat percentage at basal levels in Dlk1-/- versus Dlk1+/+ animals (Figure 2E-F). Yet, an increase in % fat was substantial for Dlk1+/+ animals on HFD induction with only a modest effect in Dlk1-/- female animals and none in males (Fig. 2E-F).

### Conclusion

Together these animal studies demonstrate that irrespective of obesity onset, mice specifically lacking Dlk1 are protected from HFD induced obesity. These data are in agreement with the human data (example 2) showing a positive association between DLK1 and fat masses.

### Example 4 - DLK1 relates to a metabolic profile associated with insulin resistance in mice and humans

### Aim of study

Since the above data revealed that a lack of Dlk1 protects against obesity, it was hypothesized that it would also protect against impaired glucose tolerance as induced by HFD.

### Results

Except for adult males, all animals on HFD displayed higher fasting blood glucose levels than their NC-fed controls (data not shown). This was independent of genotype, gender or age at onset (6- or 16-weeks of age) of diet-induced obesity (data not shown). As expected, blood glucose was significantly higher in the adult compared to the juvenile animal group (data not shown), reflecting that age decreased insulin sensitivity as expected. However, neither genotype nor gender influenced these results, suggesting that Dlk1 does not have an impact on the normal age-related negative changes in glucose homeostasis as such.

Next the animals were subjected to glucose tolerance tests (GTT) (Fig. 3A-B). Overall, GTT profiles (Fig. 3A-B) reflected that females responded to HFD by a greater reduction in glucose tolerance than males. This is in agreement with the %fat profiles described above (Fig. 2C, F), and underscores the importance of gender, at least in the experimental mouse of the HFD model. However, whereas juvenile Dlk1+/+ females, in which HFD-induced impairment of glucose clearance was obvious, Dlk1-/- animals, were protected against glucose intolerance (Fig. 3A). This phenotype was, however, attenuated in adult animals where the ability to clear glucose was similar between genotypes independent of gender (Fig. 3B). Suppressing the bioavailable DLK1 levels in vivo by 73% with an antibody in juvenile Dlk1+/+ females on HFD did not result in any acute changes in blood glucose clearance suggesting that the phenotype is due to more chronic changes. Interestingly, while fasting glucose levels were unaffected by genotype, fasting insulin levels were significantly lower in Dlk1-/- animals as compared to Dlk1+/+ controls, leading to reduced HOMA-IR levels (Fig. 3C), an estimate of insulin resistance based on fasting glucose and insulin levels. Thus, in agreement with the above data where Dlk1 enhanced obesity (example 1), it similarly has a negative impact on insulin sensitivity.

To extent these data into humans, an analysis of whole body insulin sensitivity by the gold standard technique: the euglycaemic hyperinsulinaemic clamp with DLK1 levels, was performed. Indeed, glucose disposal rates (GDR) for 48 (68 year-old (9); median BMI: 30.0 (5.6)) of the 58 previously described Danish men correlated negatively to serum DLK1 (Fig. 4A). Importantly, this correlation remained significant after multivariate regression analysis taking age, BMI and percent body fat into consideration (data not shown). Moreover, serum DLK1 was positively correlated with HOMA-IR and Adipo-IR (Fig. 4B-C). The latter indicating that insulin resistance also in adipose tissue may be affected by Dlk1 and contribute to the reduction of whole-body insulin sensitivity. As noted above, the examined cohorts herein did not have established type 2 diabetes with relative low levels of the inflammation marker MCP-1 and these did further not correlate with DLK1 (Fig. 4D). Similar results were observed for DLK1 and the Italian cohorts (Fig. 4E-G).

### Conclusion

Together these data extend the animal results into humans and indicate that increased plasma DLK1 is an early predictor of insulin resistance and TD2 in humans (at least men) before the onset of MCP-1 increase.

### Example 5 - Dlk1 knockdown in mice increases glucose uptake in skeletal muscle, but do not affect gluconeogenesis upon diet-induced obesity.

### Aim of study

To assess whether the above results could be explained by DLK1 mediating inhibition of gluconeogenesis in the liver or inhibition of skeletal muscle glucose uptake.

### Results

Measuring the level of glucagon in the NC and HFD treated juvenile female animals showed an increase upon HFD, but it did not reveal any apparent difference between genotypes. This suggests that gluconeogenesis likely is not affected in Dlk1-/- animals, and thus seems not responsible for the observed resistance to obesity induced insulin resistance (Fig. 2). In agreement, hepatic glucose production in the population of Danish men were non-correlated to DLK1 levels at neither basal or clamp conditions.

Next, the glucose uptake in skeletal muscle, using 18F-FDG PET/CT-scanning was tested (Fig. 5A). As such, female Dlk1+/+ and Dlk1-/- mice were fed only short-term (6 weeks versus 25 and 28 weeks used above, respectively) with NC or HFD, starting at 3 weeks. The standardized 18F-FDG uptake values (SUVs) for each animal (skeletal hind limb muscle vs. brain) were similar to those described by others, and all animals responded well to insulin treatment with an enhanced FDG uptake (Fig. 5B-C). Most interestingly, Dlk1-/- mice responded to the short duration of high calorie intake by increasing their skeletal muscle glucose uptake significantly (Fig. 5B), supporting a higher ability for body glucose clearance as observed above (Fig. 3A). In contrast, Dlk1+/+ animals were unaffected in their glucose uptake ability by this short-term HFD (Fig. 5B). On the molecular level, it was found that expression of the major glucose transporter Glut4 was increased in Dlk1-/- animals as compared with Dlk1+/+ controls, which was verified at the protein level (Fig. 5D-F).

### Conclusion

Lack of Dlk1 improves skeletal muscle glucose uptake by increased expression of the major glucose transporter GLUT4.

### Example 6 - Dlk1 inhibits Glut4 expression in skeletal myoblasts and preadipocytes

### Aim of study

To confirm Dlk1s impact on Glut4 expression,

### Results

Skeletal myoblast was transfected with different Dlk1 constructs and differentiated the cells into multinucleated myotubes. However, only a minor fraction of myotubes expressed DLK1 protein, and thus disabled further Glut4 evaluation. Alternatively, non-differentiated myoblasts showed a transfection efficiency of 40%, at least at time 0, and although they express low levels of Glut 4, it was attempted to evaluate Glut4 expression in these cells. Despite the limitations, indeed it was found that Glut4 was decreased in Dlk1 transfected cultures as compared to cultures transfected with empty vector (Fig. 6A), and this was further aggravated by increasing the DLK1 overexpression 10-fold (Fig. 6B). Furthermore, it was found that differentiated preadipocytes that are easily transfected and express high amounts of DLK1, to increase their Glut4 expression substantially upon siRNA mediated knock-down of endogenous Dlk1 (Fig. 6C).

### Conclusion

Together, this points to Dlk1 regulating Glut4 mediated glucose uptake, and may at least partly explain why Dlk1-/- animals are less obese and exhibit insulin resistance as compared with Dlk1+/+ mice.

### Example 7 - The correlation between DLK1 and glucose disposal rate is a direct and highly significant effect.

### Aim of study

To rule out that the association between DLK1 and GDR is mediated indirectly by fat percentage, age and/or BMI.

### Results

Serum DLK1 is negatively correlated with glucose disposal rates (GDR) for 48 (68 year-old (9); median BMI: 30.0 (5.6)) of the 58 previously described Danish men (Fig. 4A). Since DLK1 also significantly correlates with BMI and fat percentage and in turn, BMI and fat percentage correlate negatively with GDR (not shown) we speculated whether the observed DLK1-GDR correlation was simply mediated via these parameters. This was tested statistically in a mediation analysis (Fig. 7A). Importantly, no evidence of mediation of the effect of DLK1 by age, BMI, and fat percentage was observed (Fig. 7).

### Conclusion

The direct effect from DLK1 to GDR is statistically significant (p = 0.007). None of the three indirect effects explaining the extent to which the effect of DLK1 is mediated by age, BMI, and fat percentage, respectively, are statistically significant at the 0.05 significance level. The total effect is highly statistically significant (p < 0.001).

### Summary of provided data

The presented data surprisingly show that Dlk1 is not an inhibitor of in vivo fat tissue mass in healthy to overweight humans and mice. On the contrary, Dlk1 is associated with increased obesity and has a negative impact on whole body glucose homeostasis upon obesity.

Specifically, in this study it was demonstrated that absence of Dlk1 is not permissive for fat accumulation in young or adult mice, and that Dlk1-/- mice after a HFD diet indeed are resistant to obesity as compared to Dlk1+/+ animals.

The presented data suggest that Dlk1 impacts glucose uptake in skeletal muscle through regulation of the glucose transport machinery. The Dlk1 mediated Glut4 regulation was performed independent in two different cell lines, with two different gene manipulation strategies, and confirmed in vivo in Dlk1-/- mice. This suggest that the in vivo phenotype cannot solely be explained by Dlk1 impacting muscle growth during development and is further supported by the relative skeletal muscle/brain 18F-FDG PET/CT glucose uptake experiments that is independent on muscle size. Yet, since DLK1 expression is very low in normal adult skeletal muscle, it will be interesting to see whether the observed Glut4 effect is mediated by soluble DLK1, working as a hormone and part of a novel feedback axis.
It is noteworthy that increased levels of circulating DLK1 in this study were strongly associated with decreased glucose disposal rates in subjects with no diagnosed insulin resistance. Whereas MCP-1 is an often-used prognostic tool for TD2, DLK1 has never been suggested for this purpose.

## Claims

1. A method for predicting the risk of developing Insulin resistance and/or type 2 diabetes and/or metabolic syndrome for a subject, said method comprising
• determining in a biological sample from a subject the level of DLK1;
• comparing said level to a predetermined reference level;
wherein a determined level above said reference level is indicative of said subject being at risk of developing Insulin resistance and/or type 2 diabetes and/or metabolic syndrome and a determined level equal to or below said reference level is indicative of said subject not being at risk of developing Insulin resistance and/or type 2 diabetes and/or metabolic syndrome.

2. The method according to claim 1, wherein the metabolic syndrome is predicted by at least three of the five following medical conditions: abdominal obesity, high blood pressure, high blood sugar, high serum triglycerides and low high-density lipoprotein (HDL) levels.

3. The method according to claim 1 or 2, wherein the biological sample is selected from the group consisting of a blood sample, such as whole blood, serum and/or plasma, saliva, and urine, preferably blood serum or plasma.

4. The method according to any of the preceding claims, wherein the biological sample is blood serum or plasma.

5. The method according to any of the preceding claims, wherein the level of DLK1 is determined at the mRNA level and/or the protein level, preferably the protein level.

6. The method according to any preceding claim, wherein the predetermined level is above 20 ng/ml in blood serum, such as above 30 ng/ml, such as above 40 ng/ml, such as above 50 ng/ml, preferably such as above 60 ng/ml, more preferably such as above 70 ng/ml, or such as in the range 20-100 ng/ml, such as in the range 40-80 ng/ml or preferably such as in the range 60-80 ng/ml in blood serum, and more preferably in the range 70-80 ng/ml.

7. The method according to any preceding claim, wherein the predetermined reference value is based on an average of DLK1 levels in a control population not considered at risk of having Insulin resistance and/or type 2 diabetes and/or metabolic syndrome, a control population considered of having Insulin resistance and/or type 2 diabetes and/or metabolic syndrome and/or (a) sample(s) from the same subject previously obtained in time.

8. A method for determining an increased, unchanged or decreased risk of developing Insulin resistance and/or type 2 diabetes and/or metabolic syndrome, the method comprising
• determining in a first sample from a subject the level of DLK1;
• determining in a second sample from the subject the level of DLK1, wherein said second sample has been obtained at a later time point than said first sample from said subject;
wherein, a determined DLK1 level in the second sample below the determined DLK1 level in the first sample is indicative of a lowered risk of developing Insulin resistance and/or type 2 diabetes and/or metabolic syndrome;
wherein, a determined DLK1 level in the second sample above the determined DLK1 level in the first sample is indicative of an increased risk of developing Insulin resistance and/or type 2 diabetes and/or metabolic syndrome; and
wherein, a determined DLK1 level in the second sample equal to the determined DLK1 level in the first sample is indicative of an unchanged risk of developing Insulin resistance and/or type 2 diabetes and/or metabolic syndrome.

9. A method for determining the effect of a treatment protocol against having or developing Insulin resistance and/or type 2 diabetes and/or metabolic syndrome, the method comprising
• determining in a first sample from a subject the level of DLK1;
• determining in a second sample from the subject the level of DLK1;
wherein, said second sample has been obtained at a later time point than said first sample from said subject and wherein the treatment protocol has been initiated or completed between the sampling of the first sample and the second sample; and
wherein, a determined DLK1 level in the second sample below the determined DLK1 level in the first sample is indicative of the treatment protocol being efficient against having or developing Insulin resistance and/or type 2 diabetes and/or metabolic syndrome; and
wherein, a determined DLK1 level in the second sample equal to or above the determined DLK1 level in the first sample is indicative of the treatment protocol not being efficient against having or developing Insulin resistance and/or type 2 diabetes and/or metabolic syndrome.

10. The method according to claim 9, wherein the treatment protocol is selected from the group consisting of a medical treatment, exercise, and a switch in, such as a diet promoting weight loss.

11. The method according to any of the preceding claims, wherein the method is used to devise a stratified diet for a group of subjects or a personalized diet for the subject.

12. Use of DLK1 as a biomarker for predicting a subjects risk of developing Insulin resistance and/or type 2 diabetes and/or metabolic syndrome.

13. A composition comprising a DLK1 inhibitory agent for use in the prevention or treatment of Insulin resistance and/or type 2 diabetes and/or metabolic syndrome.

14. The method according to claim 13, wherein the DLK1 inhibitory agent is selected from the group consisting of DLK1 binding agents, such as selected from the group consisting of a polyclonal antibody, a monoclonal antibody, an antibody, wherein the heavy chain and the light chain are connected by a flexible linker, an Fv molecule, an antigen binding fragment, a Fab fragment, a Fab' fragment, a F(ab')₂ molecule, a fully human antibody, a humanized antibody, and a chimeric antibody.

15. The method according to claim 13, wherein the DLK1 inhibitory agent is an agent inhibiting the translation of DLK1 RNA to protein, such as a siRNA, miRNA, antisense.
